# EUROPÄISCHE PATENTSCHRIFT

(11) **EP 3 399 880 B1**
(45) Veröffentlichungstag und Bekanntmachung des Hinweises auf die Patenterteilung: **15.04.2020**
(21) Anmeldenummer: 16819540.2
(22) Anmeldetag: 20.12.2016
(51) Int. Cl.: A42B 3/16, A42B 3/30, A61F 11/14

(54) **OHRSCHÜTZER, KOMMUNIKATIONSSYSTEM UND SCHUTZHELM**
EAR PROTECTION DEVICE, COMMUNICATIONS SYSTEM AND PROTECTIVE HELMET
PROTECTEUR D'OREILLES, SYSTÈME DE COMMUNICATION ET CASQUE DE PROTECTION

(30) Priorität: 04.01.2016 DE 102016100086
(43) Veröffentlichungstag der Anmeldung: 14.11.2018
(73) Patentinhaber: Pfanner Schutzbekleidung GmbH, 6842 Koblach (AT)
(72) Erfinder: PFANNER, Anton, 6845 Hohenems (AT)
(74) Vertreter: Schumacher & Willsau
(86) Internationale Anmeldenummer: PCT/EP2016/081978
(87) Internationale Veröffentlichungsnummer: WO 2017/118571

(56) Entgegenhaltungen:
- DE-A1-102005 003 545
- DE-A1-102010 026 997
- DE-U1-202011 000 212

## Beschreibung

Die Erfindung betrifft einen Ohrschützer für einen Gehörschutz zur Befestigung an einem Schutzhelm, wobei der Ohrschützer ein Gehäuse mit einer Lagereinrichtung aufweist, über die er in einem gabelartig ausgebildeten Tragbügel schwenkbar gelagert ist. Weiterhin betrifft die Erfindung ein Kommunikationssystem sowie einen Schutzhelm.

Ein gattungsgemäßer Ohrschützer ist aus der DE 10 2010 026 997 A1 bekannt. Der Ohrschützer bildet eine Komponente eines Gehörschutzes für einen Schutzhelm, wobei die erwähnten gabelartig ausgebildeten Tragbügel über ein Kippgelenk und eine Drehplatte an dem Schutzhelm gelagert sind. Der in der DE 10 2010 026 997 A1 offenbarte Gehörschutz zeichnet sich dadurch aus, dass die Ohrschützer in eine Parkposition innerhalb des Helms verschwenkbar sind.
Da der Gehörschutz eines Schutzhelms nicht nur erwünschten Schall davon abhält, an das Ohr des Trägers vorzudringen, wie zum Beispiel Lärm von Werkzeugen und Maschinen, sondern auch die Kommunikation zwischen den Helmträgern erschwert, ist es nützlich, Kommunikationsmittel bereitzustellen, die es auch bei in Arbeitsstellung befindlichem Gehörschutz ermöglichen, dass die mit dem Schutzhelmen und dem Gehörschutz geschützten Personen miteinander kommunizieren können. Davon soll die schützende Wirkung des Gehörschutzes nicht negativ beeinflusst werden. Die DE 10 2005 003 545 A1 und DE 20 2011 000 212 U1 beschreiben Ohrschützer zur Befestigung an einem Schutzhelm die eine Kommunikationseinrichtung enthalten, gemäß dem Oberbegriff des Anspruchs 1.

Es ist eine Aufgabe der Erfindung, einen Ohrschützer in der Weise weiterzubilden, dass er eine Kommunikation ermöglicht, wobei seine schützende Wirkung erhalten bleibt. Dabei soll insbesondere auf störende Kabelverbindungen verzichtet werden können.

Diese Aufgabe wird mit den Merkmalen des unabhängigen Anspruches gelöst. Vorteilhafte Ausführungsformen der Erfindung sind in den unter- und nebengeordneten Ansprüchen angegeben.

Die Erfindung baut auf den gattungsgemäßen Ohrschützer dadurch auf, dass in und/oder an dem Gehäuse eine Kommunikationseinrichtung angeordnet ist und dass die Lagereinrichtung mindestens zwei elektrisch leitfähige Kontaktelemente aufweist. Das Gehäuse des Ohrschützers hat insofern eine mehrfache Funktion. Zum einen bildet es die formstabile Komponente des Ohrschützers, es dient über seine Lagereinrichtung dem schwenkbaren Lagern des Ohrschützers im Tragbügel, es beherbergt eine Kommunikationseinrichtung und ist ferner Träger elektrisch leitfähiger Kontaktelemente, die für den Betrieb der Kommunikationseinrichtung wesentlich sind.

Die Erfindung ist besonders nützlich dadurch weitergebildet, dass die Lagerreinrichtung zwei einstückig mit dem Gehäuse ausgebildete Zapfen aufweist und dass die elektrisch leitfähigen Kontaktelemente aus Öffnungen der Zapfen herausragen, welche ein Gehäuseinneres und ein Gehäuseäußeres miteinander verbinden. Diese Konstruktion lässt sich einfach dadurch herstellen, dass Löcher in die als Zapfen ausgebildete Lagereinrichtung, die einstückig mit dem Gehäuse des Ohrschützers ausgebildet ist, gebohrt werden.

Nützlicherweise ist vorgesehen, dass die Kontaktelemente die Öffnungen der Zapfen staub- und/oder flüssigkeitsdicht verschließen. Hierdurch werden die elektrischen Komponenten innerhalb des Ohrschützergehäuses gegen schädliche Umwelteinflüsse geschützt.

Gemäß einer bevorzugte Ausführungsform der Erfindung kann vorgesehen sein, dass die Kommunikationseinrichtung einen Lautsprecher umfasst, der in dem Ohrschützer angeordnet ist. Hierdurch wird er Träger des Ohrschützers in die Lage versetzt, über das Kommunikationssystem übertragene Informationen über sein Ohr zu empfangen.

Ebenfalls ist es besonders nützlich, dass die Kommunikationseinrichtung ein Mikrophon umfasst, das über eine Mikrophonhalterung an dem Ohrschützer angeordnet ist. Das Mikrophon ermöglicht dem Träger des Ohrschützers, Informationen an die Kommunikationseinrichtung zu übermitteln.

In diesem Zusammenhang ist es besonders nützlich, dass die Kommunikationseinrichtung eine Sende-Empfangs-Einrichtung aufweist. Die Kommunikationseinrichtung kann auf dieser Grundlage von außen übertragene Signale empfangen und die entsprechenden Informationen über den Lautsprecher im Ohrschützer an den Träger des Ohrschützers weitergeben. Umgekehrt empfängt die Kommunikationseinrichtung Signale vom Mikrophon, das mit der Kommunikationseinrichtung gekoppelt ist. Entsprechende Signale, die von externen Kommunikationseinrichtungen empfangen werden können, werden dann von der Sende-Empfangs-Einrichtung der internen Kommunikationseinrichtung ausgegeben.

Die Erfindung ist in besonders vorteilhafter Weise dadurch weitergebildet, dass die Sende-Empfangs-Einrichtung ein Bluetooth-Transceiver ist. Bei Bluetooth handelt es sich um einen Industriestandard für die Datenübertragung zwischen Geräten über kurze Distanz per Funktechnik. Die Übertragung ist bidirektional und störungsunanfällig, wodurch das Kommunikationssystem im Zusammenhang mit dem erfindungsgemäßen Ohrschützer besonders komfortabel ausgestaltet ist. Ferner wird hierdurch ein hohes Maß an Sicherheit zur Verfügung gestellt, wodurch der erfindungsgemäße Ohrschützer die von dem Schutzhelm gelieferte Sicherheit unterstützt.

Gemäß einer bevorzugten Ausführungsform der Erfindung ist diese dadurch weitergebildet, dass in dem Gehäuse ein Akkumulator angeordnet ist, der die Kommunikationseinrichtung mit Energie versorgt und der über die elektrisch leitfähigen Kontaktelemente aufladbar ist. Der Ohrschützer kann also in sich mit allen Komponenten ausgestattet sein, die eine Kommunikationseinrichtung benötigt. Insbesondere ist der Akkumulator als temporäre Energiequelle innerhalb des Ohrschützers vorgesehen. Die elektrisch leitfähigen Kontaktelemente am Ohrschützer müssen somit nur die Funktion von Ladekontakten zur Verfügung stellen.

Nützlicherweise ist vorgesehen, dass an dem Gehäuse Bedienelemente und/oder Anzeigeelemente zum Steuern und/oder Überwachen der Kommunikationseinrichtung vorgesehen sind. Als Bedienelemente können insbesondere ein Taster zum Ein- und Ausschalten der Kommunikationseinrichtung vorgesehen sein. Dieser Taster kann weiterhin dazu dienen, den Bluetooth-Transceiver mit anderen Bluetooth-Transceivern zu koppeln, beispielsweise mit solchen, die in Ohrschützern anderer Träger angeordnet sind, oder auch mit Mobiltelefonen oder Kraftfahrzeug-Transceivern. Selbstverständlich kann hierzu auch ein eigens hierfür vorgesehener Taster am Gehäuse angeordnet sein. Weitere Bedienelemente können zum Ändern der Lautstärke der von dem Lautsprecher abgegebenen akustischen Signale vorgesehen sein. Ebenfalls können Bedienelemente dem Einstellen der Mikrophonempfindlichkeit dienen. Als Anzeigeelement können im einfachsten Fall eine oder mehrere Leuchtdioden vorgesehen sein, die durch Lichtsignale an sich, die Farbe der Lichtsignale und/oder die zeitliche Abfolge von Lichtsignalen Informationen an den Nutzer des Ohrschützers übermitteln können.

Die Erfindung betrifft ferner eine Kommunikationseinrichtung mit einem erfindungsgemäßen Ohrschützer und mit einem gabelartig ausgebildeten Tragbügel, in dem der Ohrschützer schwenkbar gelagert ist, wobei der Tragbügel eine elektrische Verbindung zu den elektrisch leitfähigen Kontaktelementen zur Verfügung stellt.

Dabei kann vorgesehen sein, dass der Tragbügel eine von einem Schutzhelm unabhängige Andockstation ist. Die so bereitgestellte Andockstation kann beispielsweise in einem Kraftfahrzeug, in einem Zubehörkoffer oder an beliebiger sonstiger Stelle zur Verfügung gestellt werden. Insbesondere kann die Andockstation stationär oder mobil ausgestaltet sein.

Nützlicherweise kann vorgesehen sein, dass die Andockstation von Solarzellen mit Energie versorgt wird. Die Bereitstellung der Energie durch Solarzellen macht den Träger des Schutzhelms unabhängig von klassischen Energiequellen. Gerade in Situationen, die einen Schutzhelm erforderlich machen, beispielsweise bei Forstarbeit oder auf Baustellen, stehen Energiequellen nicht ohne weiteres zur Verfügung, so dass die Lieferung der Energie über Solarzellen von besonderem Vorteil ist.

Neben der Möglichkeit, dass der Tragbügel eine von dem Schutzhelm unabhängige Andockstation ist, besteht auch die Möglichkeit, dass der Tragbügel an einem Schutzhelm befestigbar ist und gleichzeitig Andockstation sowie Tragbügel zur Verwendung während des Einsatzes des Schutzhelms ist. In diesem Fall kann es vorgesehen sein, dass der Tragbügel insgesamt zusammen mit dem Ohrschützer vom Helm entfernt wird, um diesen dann mit einer Energiequelle zu verbinden. Ebenfalls ist es denkbar, dass ein Akkumulator an beliebiger Stelle des Helms untergebracht ist, wobei die für den Betrieb des Bluetooth-Transceivers erforderliche Energie dann während des Einsatzes des Schutzhelms über die elektrischen Kontaktelemente des Ohrschützers an den Bluetooth-Transceiver geliefert wird. Ebenfalls ist es möglich, den Schutzhelm mit einer elektrischen Schnittstelle auszustatten, über die Ladestrom und/oder Daten an den Ohrschützer übergeben werden können.

Die Erfindung betrifft weiterhin einen Schutzhelm mit mindestens einem gabelartigen Tragbügel zum Lagern eines erfindungsgemäßen Ohrschützers, wobei mindestens ein Tragbügel gleichzeitig Andockstation sowie Tragbügel zur Verwendung während des Einsatzes des Schutzhelms ist.

In diesem Zusammenhang kann es nützlich sein, dass der Schutzhelm mit einem Akkumulator ausgestattet ist, durch den eine in und/oder an dem Ohrschützer angeordnete Kommunikationsvorrichtung mit Energie versorgbar ist.

Weiterhin kann es von Vorteil sein, dass der Schutzhelm eine Schnittstelle aufweist, über die ihm Energie zuführbar ist.

Die Erfindung wird nun mit Bezug auf die begleitenden Zeichnungen anhand besonders bevorzugter Ausführungsformen beispielhaft erläutert.
- Figur 1: eine Seitenansicht eines Schutzhelms mit einem erfindungsgemäßen Ohrschützer, aufgesetzt auf den Kopf eines Trägers;
- Figur 2: eine Seitenansicht eines erfindungsgenäßen Ohrschützers;

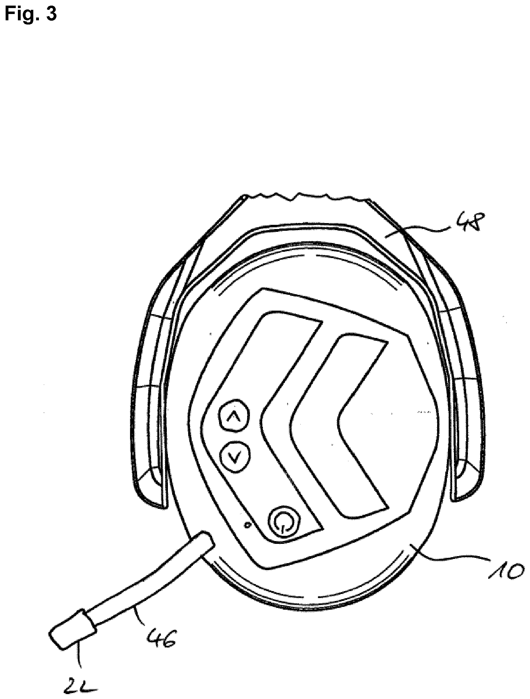

- Figur 3: eine Seitenansicht eines erfindungsgemäßen Ohrschützers in einem Tragbügel;

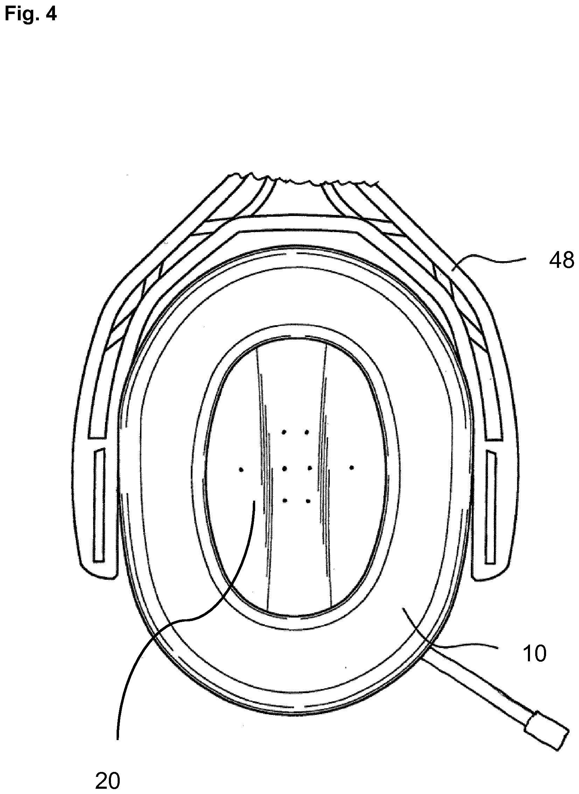

- Figur 4: eine Innenansicht eines erfindungsgemäßen Ohrschützers in einem Tragbügel;

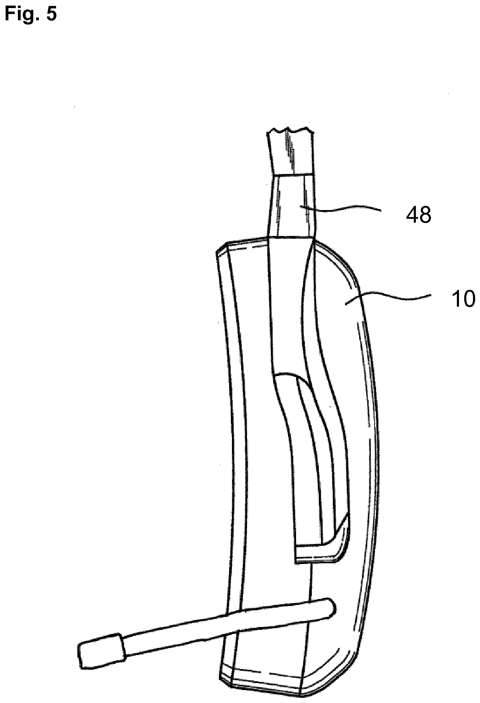

- Figur 5: eine Ansicht, bezogen auf die normale Tragweise eines Schutzhelms von vorne, auf einen erfindungsgemäßen Ohrschützer mit Tragbügel;

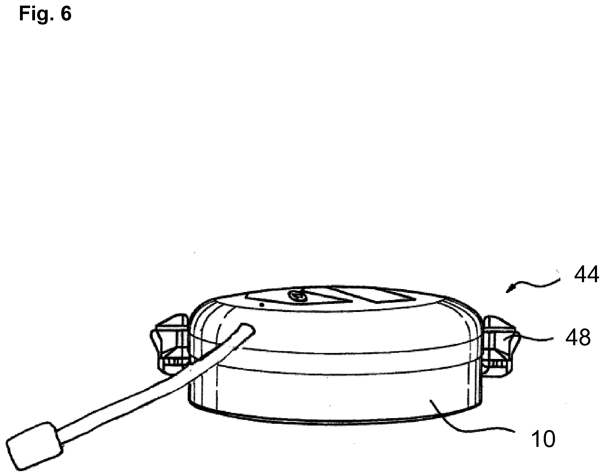

- Figur 6: eine Ansicht, bezogen auf die normale Tragweise eines Schutzhelms von unten, auf einen erfindungsgemäßen Ohrschützer mit Tragbügel;

Bei der nachfolgenden Beschreibung der Zeichnungen bezeichnen gleiche Bezugszeichen gleiche oder vergleichbare Komponenten.

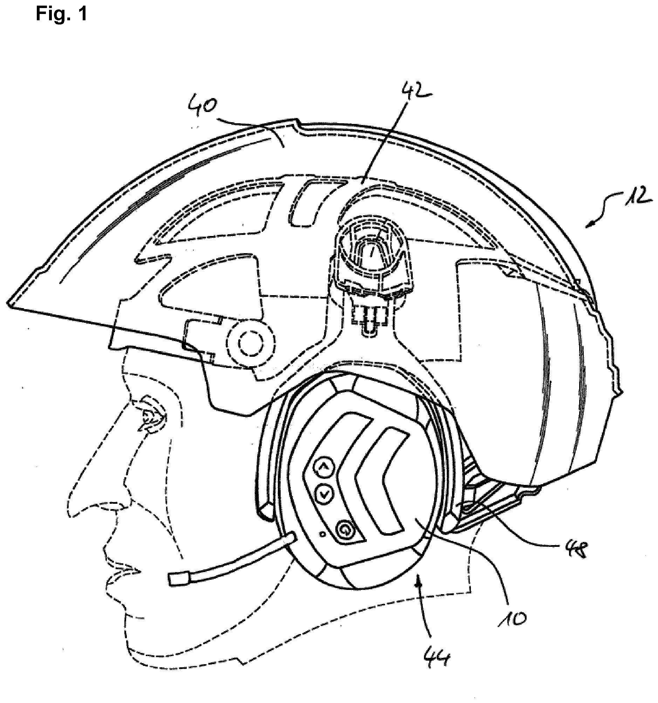

Figur 1 zeigt einen Schutzhelm 12 auf dem Kopf eines Trägers. Dieser umfasst unter anderem eine Helmschale 40, einen Tragkorb 42 sowie einen Gehörschutz 44. Der Gehörschutz 44 umfasst mindestens einen Ohrschützer 10, der in erfindungsgemäßer Weise mit einer Kommunikationseinrichtung 20, 22, 24, 26 ausgestattet ist. Der Kommunikationseinrichtung 20, 22, 24, 26 können zahlreiche Komponenten des Ohrschützers 10 zugeordnet werden, insbesondere ein Lautsprecher 20, ein Mikrophon 22, eine Sende-Empfangs-Einrichtung 24, insbesondere ein Bluetooth-Transceiver, sowie ein Akkumulator 26. Diese Komponenten sind am oder in einem Gehäuse 14 des Ohrschützers 10 angeordnet. Der Ohrschützer 10 wird von einem Tragbügel 48 gehalten.

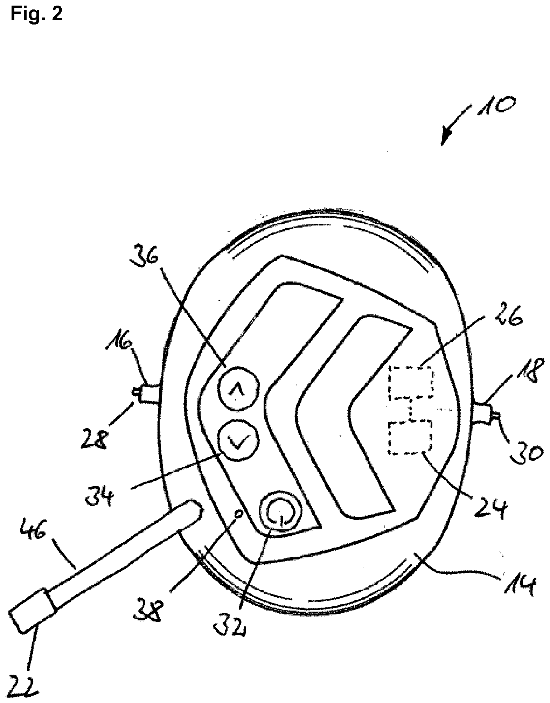

In Figur 2 ist mit unterbrochenen Linien angedeutet, dass sich die Sende-Empfangs-Einrichtung 24 sowie der Akkumulator 26 innerhalb des Gehäuses 14 des Ohrschützers 10 befinden. Das Mikrophon 22 ist über eine Mikrophonhalterung 46 außerhalb des Gehäuses 14 des Ohrschützers 10 angeordnet. An der Außenseite des Gehäuses 14 des Ohrschützers 10 sind Bedienelemente 32, 34, 36 und ein Anzeigeelement 38 angeordnet. Bei den Bedienelementen 32, 34, 36 handelt es sich um Taster. Das Anzeigeelement 38 ist eine Leuchtdiode. Einer der Taster 32 dient dem Einschalten der Sende-Empfangs-Einrichtung 24 sowie, in dem Falle dass es sich bei der Sende-Empfangs-Einrichtung um einen Bluetooth-Transceiver handelt, zum Paaren beziehungsweise Koppeln des Bluetooth-Transceivers mit externen anderen Bluetooth-Transceivern. Weitere Taster 34, 36 sind zum Verändern der Lautstärke des Lautsprechers 20 des Ohrschützers 10 vorgesehen. An dem Gehäuse 14 des Ohrschützers 10 ist weiterhin eine Lagereinrichtung 16, 18 vorgesehen, die vorzugsweise durch einstückig mit dem Gehäuse 14 ausgebildete Zapfen 16, 18 realisiert ist. Diese Zapfen 16, 18 sind mit zentralen Bohrungen ausgestattet, aus denen elektrisch leitfähige Kontaktelemente 28, 30 vorstehen, die ein Gehäuseinneres und ein Gehäuseäußeres miteinander verbinden. Dabei schließen diese die Öffnungen der Zapfen 16, 18 staub- und/oder flüssigkeitsdicht ab. Zum elektrischen Koppeln des Ohrschützers 10 mit insbesondere einer elektrischen Energiequelle wird dieser in einen gabelartigen Tragbügel 48 eingesetzt. Dieser Tragbügel 48 kann eine von dem Schutzhelm 12 unabhängige Andockstation sein. Allerdings kann auch vorgesehen sein, dass der Tragbügel gleichzeitig Andockstation sowie Tragbügel zur Verwendung während des Einsatzes des Schutzhelms 12 ist. Jedenfalls stellt der Tragbügel 48 elektrische Kontakte zur Verfügung, die den elektrischen Kontaktelementen 28, 30 des Ohrschützers 14 zugeordnet sind. Hierüber wird die elektrische Energie zum Aufladen des Akkumulators 26 innerhalb des Gehäuses 14 des Ohrschützers 10 geliefert. Ebenfalls ist es möglich, dass die elektrischen Kontaktelemente zur Datenübertragung dienen. Dies kann zum Beispiel dann vorgesehen sein, wenn eine Sende-Empfangs-Einrichtung nicht innerhalb des Gehäuses 14 angeordnet ist, sondern außerhalb. Eine weitere Möglichkeit besteht darin, dass die elektrischen Kontaktelemente 28, 30 zwar der Energieübernahme durch Komponenten innerhalb des Ohrschützers 10 dienen, wobei ein Akkumulator jedoch außerhalb des Ohrschützers 10 angeordnet ist.

Mit dem so bereitgestellten Kommunikationssystem können verschiedene Kommunikationsbetriebsarten realisiert werden. Beispielsweise ist es möglich, zwei Ohrschützer mit den entsprechenden Kommunikationssystemen direkt zu koppeln, so dass eine direkte Kommunikation zwischen den Ohrschützern beziehungsweise den Trägern der Ohrschützer über die Ohrschützer erfolgen kann. Ferner können die Ohrschützer mit beliebigen Sende-Empfangs-Einrichtungen gekoppelt werden, beispielsweise über die Bluetooth-Funktion eines Kraftfahrzeugs oder eines Mobiltelefons. Insbesondere bei der Kopplung mit einem Mobiltelefon kann der Träger des Ohrschützers Telefonate einleiten beziehungsweise annehmen und dabei den Ohrschützer nutzen. Ebenfalls ist es möglich, die Kommunikationsreichweite zwischen zwei Trägern von Ohrschützern zu vergrößern. Einerseits ist dies selbstverständlich dadurch möglich, dass beide Ohrschützer mit unterschiedlichen Telefonen gekoppelt sind und die Kommunikation über eine Telefonverbindung zwischen den beiden Telefonen und jeweilige Bluetooth-Datenverbindungen zwischen den Telefonen und den Ohrschützern erfolgt. Es ist aber auch möglich, beispielsweise ein Mobiltelefon mit zwei Ohrschützern zu koppeln. Befindet sich dieses Mobiltelefon in der Reichweite beider Ohrschützer, so können die Träger der jeweiligen Ohrschützer miteinander kommunizieren, obgleich sie eine solche Entfernung von einander aufweisen, dass eine direkte Kommunikation zwischen ihnen nicht mehr möglich wäre.

Die in der vorstehenden Beschreibung, in den Zeichnungen sowie in den Ansprüchen offenbarten Merkmale der Erfindung können sowohl einzeln als auch in beliebiger Kombination für die Verwirklichung der Erfindung wesentlich sein.

### Bezugszeichenliste

- 10: Ohrschützer
- 12: Schutzhelm
- 14: Gehäuse
- 16: Lagereinrichtung/Zapfen
- 18: Lagereinrichtung/Zapfen
- 20: Lautsprecher
- 22: Mikrophon
- 24: Sende-Empfangs-Einrichtung
- 26: Akkumulator
- 28: Kontaktelement
- 30: Kontaktelement
- 32: Taster
- 34: Taster
- 36: Taster
- 38: Anzeigeelement
- 40: Helmschale
- 42: Tragkorb
- 44: Gehörschutz
- 46: Mikrophonhalterung
- 48: Tragbügel

## Patentansprüche

1. Ohrschützer (10) für einen Gehörschutz zur Befestigung an einem Schutzhelm (12), wobei der Ohrschützer (10) ein Gehäuse (14) mit einer Lagereinrichtung (16, 18) aufweist, über die er in einem gabelartig ausgebildeten Tragbügel (48) schwenkbar lagerbar ist, wobei in und/oder an dem Gehäuse (14) eine Kommunikationseinrichtung (20, 22, 24, 26) angeordnet ist, **dadurch gekennzeichnet, dass** die Lagereinrichtung mindestens zwei elektrisch leitfähige Kontaktelemente (28, 30) aufweist.

2. Ohrschützer (10) nach Anspruch 1, **dadurch gekennzeichnet, dass** die Lagerreinrichtung (16, 18) zwei einstückig mit dem Gehäuse ausgebildete Zapfen (16, 18) aufweist und dass die elektrisch leitfähigen Kontaktelemente (28, 30) aus Öffnungen der Zapfen (16, 18) herausragen, welche ein Gehäuseinneres und ein Gehäuseäußeres miteinander verbinden.

3. Ohrschützer (10) nach Anspruch 2, **dadurch gekennzeichnet, dass** die Kontaktelemente (28, 30) die Öffnungen der Zapfen (16, 18) staub- und/oder flüssigkeitsdicht verschließen.

4. Ohrschützer (10) nach einem der vorhergehenden Ansprüche, **dadurch gekennzeichnet, dass** die Kommunikationseinrichtung (20, 22, 24, 26) einen Lautsprecher (20) umfasst, der in dem Ohrschützer angeordnet ist.

5. Ohrschützer (10) nach einem der vorhergehenden Ansprüche, **dadurch gekennzeichnet, dass** die Kommunikationseinrichtung (20, 22, 24, 26) ein Mikrophon (22) umfasst, das über eine Mikrophonhalterung (46) an dem Ohrschützer angeordnet ist.

6. Ohrschützer (10) nach einem der vorhergehenden Ansprüche, **dadurch gekennzeichnet, dass** die Kommunikationseinrichtung (20, 22, 24, 26) eine Sende-Empfangs-Einrichtung (24) aufweist.

7. Ohrschützer (10) nach Anspruch 6, **dadurch gekennzeichnet, dass** die Sende-Empfangs-Einrichtung (24) ein Bluetooth-Transceiver ist.

8. Ohrschützer (10) nach einem der vorhergehenden Ansprüche, **dadurch gekennzeichnet, dass** in dem Gehäuse ein Akkumulator (26) angeordnet ist, der die Kommunikationseinrichtung (20, 22, 24, 26) mit Energie versorgt und der über die elektrisch leitfähigen Kontaktelemente (28, 30) aufladbar ist.

9. Ohrschützer (10) nach einem der vorhergehenden Ansprüche, **dadurch gekennzeichnet, dass** an dem Gehäuse (14) Bedienelemente (32, 34, 36) und/oder Anzeigeelemente (38) zum Steuern und/oder Überwachen der Kommunikationseinrichtung vorgesehen sind.

10. Kommunikationssystem mit einem Ohrschützer (10) nach einem der vorhergehenden Ansprüche und einem gabelartig ausgebildeten Tragbügel (48), in dem der Ohrschützer (10) schwenkbar gelagert ist, wobei der Tragbügel (48) eine elektrische Verbindung zu den elektrisch leitfähigen Kontaktelementen (28, 30) zur Verfügung stellt.

11. Kommunikationssystem nach Anspruch 10, **dadurch gekennzeichnet, dass** der Tragbügel (48) eine von einem Schutzhelm (12) unabhängige Andockstation ist.

12. Kommunikationssystem nach Anspruch 10, **dadurch gekennzeichnet, dass** die Andockstation von Solarzellen mit Energie versorgt wird.

13. Kommunikationssystem nach Anspruch 10, **dadurch gekennzeichnet, dass** der Tragbügel (48) an einem Schutzhelm (12) befestigbar ist und gleichzeitig Andockstation sowie Tragbügel zur Verwendung während des Einsatzes des Schutzhelms (12) ist.

14. Schutzhelm (12) mit mindestens einem gabelartigen Tragbügel (48) und einem im gabelartigen Tragbügel (48) gelagerten Ohrschützer (10) nach einem der Ansprüche 1 bis 9, wobei mindestens ein Tragbügel (48) gleichzeitig Andockstation sowie Tragbügel zur Verwendung während des Einsatzes des Schutzhelms ist.

15. Schutzhelm (12) nach Anspruch 14, **dadurch gekennzeichnet, dass** der Schutzhelm (12) mit einem Akkumulator (26) ausgestattet ist, durch den eine in und/oder an dem Ohrschützer (10) angeordnete Kommunikationseinrichtung (20, 22, 24, 26) mit Energie versorgbar ist.

16. Schutzhelm (12) nach Anspruch 14 oder 15, **dadurch gekennzeichnet, dass** der Schutzhelm (12) eine Schnittstelle aufweist, über die ihm Energie zuführbar ist.

## Claims

1. An ear protection device (10) for ear defenders to be fastened on a protective helmet (12), wherein the ear protection device (10) comprises a housing (14) including a bearing device (16, 18) via which it is pivotably mountable in a forklike supporting bracket (48), wherein communication means (20, 22, 24, 26) are disposed in and/or on the housing (14), **characterised in that** the bearing device includes at least two electrically conductive contact elements (28, 30).

2. The ear protection device (10) according to claim 1, **characterised in that** the bearing device (16, 18) includes two spigots (16, 18) integrally formed with the housing and **in that** the electrically conductive contact elements (28, 30) protrude from openings of the spigots (16, 18) connecting an interior of the housing with an exterior of the housing.

3. The ear protection device (10) according to claim 2, **characterised in that** the contact elements (28, 30) close the openings of the spigots (16, 18) so as to be dust- and/or liquid-tight.

4. The ear protection device (10) according to one of the preceding claims, **characterised in that** the communication means (20, 22, 24, 26) comprise a loudspeaker (20) disposed in the ear protection device.

5. The ear protection device (10) according to one of the preceding claims, **characterised in that** the communication means (20, 22, 24, 26) comprise a microphone (22) disposed on the ear protection device via a microphone attachment (46).

6. The ear protection device (10) according to one of the preceding claims, **characterised in that** the communication means (20, 22, 24, 26) include a transceiver device (24).

7. The ear protection device (10) according to claim 6, **characterised in that** the transceiver device (24) is a Bluetooth transceiver.

8. The ear protection device (10) according to one of the preceding claims, **characterised in that** a battery (26) supplying the communication means (20, 22, 24, 26) with power and being rechargeable via the electrically conductive contact elements (28, 30) is disposed in the housing.

9. The ear protection device (10) according to one of the preceding claims, **characterised in that** control elements (32, 34, 36) and/or display elements (38) for controlling and/or monitoring the communication means are provided on the housing (14).

10. A communications system comprising an ear protection device (10) according to one of the preceding claims and a forklike supporting bracket (48) in which the ear protection device (10) is pivotably mounted, wherein the supporting bracket (48) provides for an electric connection to the electrically conductive contact elements (28, 30).

11. The communications system according to claim 10, **characterised in that** the supporting bracket (48) is a docking station independent of a protective helmet (12).

12. The communications system according to claim 10, **characterised in that** the docking station is supplied with power by photovoltaic cells.

13. The communications system according to claim 10, **characterised in that** the supporting bracket (48) is attachable to a protective helmet (12) and is both a docking station and a supporting bracket for use while the protective helmet (12) is in use.

14. A protective helmet (12) comprising at least one forklike supporting bracket (48) and an ear protection device (10) supported in the forklike supporting bracket (48) according to one of the claims 1 to 9, wherein at least one supporting bracket (48) is both a docking station and a supporting bracket for use while the protective helmet is in use.

15. The protective helmet (12) according to claim 14, **characterised in that** the protective helmet (12) is equipped with a battery (26) by means of which a communication means (20, 22, 24, 26) disposed in and/or on the ear protection device (10) can be supplied with power.

16. The protective helmet (12) according to claim 14 or 15, **characterised in that** the protective helmet (12) comprises an interface via which it can be supplied with power.

## Revendications

1. Protection d'oreilles (10) pour une protection auditive à fixer sur un casque de protection (12), la protection d'oreilles (10) présentant une enveloppe (14) avec un dispositif de logement (16, 18) grâce auquel elle peut être placée de manière pivotante dans un étrier de support (48) en forme de fourche, un système de communication (20, 22, 24, 26) étant disposé dans et/ou sur l'enveloppe (14), **caractérisée en ce que** le dispositif de logement présente au moins deux éléments de contact (28, 30) conducteurs d'électricité.

2. Protection d'oreilles (10) selon la revendication 1, **caractérisée en ce que** le dispositif de logement (16, 18) présente deux ergots (16, 18) formés d'une seule pièce avec l'enveloppe et **en ce que** les éléments de contact (28, 30) conducteurs d'électricité dépassent les ergots (16, 18) qui relient l'intérieur de l'enveloppe à l'extérieur de l'enveloppe.

3. Protection d'oreilles (10) selon la revendication 2, **caractérisée en ce que** les éléments de contact (28, 30) ferment de façon étanche aux liquides et à la poussière les ouvertures des ergots (16, 18).

4. Protection d'oreilles (10) selon l'une des revendications précédentes, **caractérisée en ce que** le système de communication (20, 22, 24, 26) comprend un haut-parleur (20) qui est disposé dans la protection d'oreilles.

5. Protection d'oreilles (10) selon l'une des revendications précédentes, **caractérisée en ce que** le système de communication (20, 22, 24, 26) comprend un microphone (22) qui est disposé dans la protection d'oreilles sur un support pour microphone (46).

6. Protection d'oreilles (10) selon l'une des revendications précédentes, **caractérisée en ce que** le système de communication (20, 22, 24, 26) présente un dispositif émetteur-récepteur (24).

7. Protection d'oreille (10) selon la revendication 6, **caractérisée en ce que** le dispositif émetteur-récepteur (24) est un émetteur-récepteur Bluetooth.

8. Protection d'oreilles (10) selon l'une des revendications précédentes, **caractérisée en ce qu'un** accumulateur (26) est disposé dans l'enveloppe, accumulateur qui alimente le système de communication (20, 22, 24, 26) en énergie et qui peut être rechargé par les éléments de contact (28, 30) conducteurs d'électricité.

9. Protection d'oreilles (10) selon l'une des revendications précédentes, **caractérisée en ce que** des éléments de commande (32, 34, 36) et/ou des éléments d'affichage (38) sont prévus sur l'enveloppe pour commander et/ou surveiller le système de communication.

10. Système de communication doté d'une protection d'oreilles (10) selon l'une des revendications précédentes et un étrier de support (48) en forme de fourche dans lequel la protection d'oreilles est placée de manière pivotante, l'étrier de support (48) fournissant une liaison électrique pour les éléments de contact (28, 30) conducteurs d'électricité.

11. Système de communication selon la revendication 10, **caractérisé en ce que** l'étrier de support (48) est une station d'accueil indépendante d'un casque de protection (12).

12. Système de communication selon la revendication 10, **caractérisé en ce que** la station d'accueil est alimentée en énergie par des cellules solaires.

13. Système de communication selon la revendication 10, **caractérisé en ce que** l'étrier de support (48) peut être fixé à un casque de protection (12) et est simultanément une station d'accueil ainsi qu'un étrier de support pour être utilisés avec le casque de protection (12).

14. Casque de protection (12) avec au moins un étrier de support (48) en forme de fourche et une protection d'oreilles (10) logée dans l'étrier de support (48) selon l'une des revendications 1 à 9, au moins un étrier de support (48) étant simultanément une station d'accueil ainsi qu'un étrier de support pour être utilisés avec le casque de protection (12).

15. Casque de protection (12) selon la revendication 14, **caractérisé en ce que** le casque de protection (12) est doté d'un accumulateur (26) grâce auquel le système de communication (20, 22, 24, 26) disposé dans et/ou sur la protection d'oreilles (10) peut être alimenté en énergie.

16. Casque de protection (12) selon la revendication 14 ou 15, **caractérisé en ce que** le casque de protection (12) présente une interface par laquelle de l'énergie peut lui être alimentée.
